# FASCICULE DE BREVET EUROPEEN

(11) **EP 3 728 408 B1**
(45) Date de publication et mention de la délivrance du brevet: **06.10.2021**
(21) Numéro de dépôt: 18811273.4
(22) Date de dépôt: 04.12.2018
(51) Int. Cl.: C08H 8/00

(54) **PROCEDE DE TRAITEMENT DE BIOMASSE LIGNO-CELLULOSIQUE PAR IMPREGNATION**
VERFAHREN ZUR BEHANDLUNG VON LIGNOZELLULOSEHALTIGER BIOMASSE DURCH IMPRÄGNIERUNG
METHOD FOR TREATING LIGNOCELLULOSIC BIOMASS BY IMPREGNATION

(30) Priorité: 20.12.2017 FR 1762611
(43) Date de publication de la demande: 28.10.2020
(73) Titulaire: IFP Energies nouvelles, 92852 Rueil-Malmaison (FR); Agro Industries Recherche Et Developpement, 51110 Pomacle (FR); Institut national de recherche pour l'agriculture, l'alimentation et l'environnement, 75338 Paris Cedex 07 (FR)
(72) Inventeur: AYMARD, Caroline, 69007 LYON (FR); BOUILLON, Pierre-Antoine, 69003 LYON (FR); ROUSSET, Romain, 69600 OULLINS (FR); CARNNOT, Olivier, 51400 BACONNES (FR)
(74) Mandataire: IFP Energies nouvelles
(86) Numéro de dépôt international: PCT/EP2018/083540
(87) Numéro de publication internationale: WO 2019/120994

(56) Documents cités:
- WO-A1-2010/121367
- WO-A1-2012/061939
- WO-A1-2012/088108
- WO-A1-2015/173226
- WO-A1-2018/015227
- US-A1- 2011 318 796

## Description

### Domaine de l'invention

L'invention concerne un procédé de traitement de biomasse lignocellulosique au moyen de liqueur acide pour produire des jus sucrés dit de seconde génération (2G). Ces jus sucrés peuvent être utilisés pour produire d'autres produits par voie biochimique (ex : des alcools comme l'éthanol, le butanol ou d'autres molécules, par exemple des solvants tels que l'acétone etc...). Ce procédé comprend l'analyse des flux de liquides soutirés au cours du procédé et l'ajustement de la composition de la liqueur d'imprégnation de manière à maintenir constant le pouvoir acide desdits flux.

### Art antérieur

La biomasse lignocellulosique représente une des ressources renouvelables les plus abondantes sur terre. Les substrats considérés sont très variés, ils concernent à la fois des substrats ligneux comme différents bois (feuillus et résineux), des coproduits issus de l'agriculture (pailles de blé, rafles de maïs, etc...) ou d'autres industries agroalimentaires, papetières, etc...

Le procédé de transformation biochimique de la lignocellulosique en jus sucrés 2G comprend notamment une étape de prétraitement et une étape d'hydrolyse par un cocktail enzymatique. Ces procédés comportent aussi le plus souvent une étape d'imprégnation avant le prétraitement. Les jus sucrés issus de l'hydrolyse enzymatique sont ensuite traités, par exemple par fermentation, et le procédé comprend également des étapes de séparation et/ou une étape de purification du produit final.

La biomasse lignocellulosique est composée de trois principaux polymères : la cellulose (35 à 50%), qui est un polysaccharide essentiellement constitué d'hexoses ; l'hémicellulose (20 à 30%), qui est un polysaccharide essentiellement constitué de pentoses et d'hexoses ; et la lignine (15 à 25%), qui est un polymère de structure complexe et de haut poids moléculaire, composé d'alcools aromatiques reliés par des liaisons éther. Ces différentes molécules sont responsables des propriétés intrinsèques de la paroi végétale et s'organisent en un enchevêtrement complexe. Outre ces trois composés majoritaires, la biomasse lignocellulosique contient aussi notamment des groupements acétyls et des cendres. Ces « cendres » sont des minéraux : silice, composés contenant du calcium, du magnésium, du sodium, du potassium, du phosphore et/ou de l'aluminium. Leur teneur et leur composition varie énormément d'un type de biomasse à l'autre (paille versus bois, etc..), mais aussi en fonction des conditions pédoclimatiques de culture, et des conditions de récoltes. Différents lots de paille auront des natures de cendres variables dans des teneur différentes. Une méthode pour quantifier les cendres de produits lignocellulosiques est par exemple décrite dans la norme ASTM E1755 « Standard Test Method for Ash in Biomass ».

Parmi les trois polymères de base qui intègrent la biomasse lignocellulosique, la cellulose et l'hémicellulose sont ceux qui permettent la production de jus sucrés 2G.

Le plus souvent, l'hémicellulose est majoritairement décomposée en sucres durant le prétraitement et la cellulose est convertie en glucose par l'hydrolyse enzymatique. Toutefois, l'accès à la cellulose brute reste difficilement accessibles aux enzymes d'où la nécessité d'un prétraitement. Ce prétraitement permet de modifier les propriétés physico-chimiques de la lignocellulosique afin d'améliorer l'accessibilité de la cellulose aux enzymes et sa réactivité à l'hydrolyse enzymatique.

Un des prétraitements le plus efficace est l'explosion à la vapeur en conditions acides qui permet une hydrolyse presque totale de l'hémicellulose et une amélioration importante de l'accessibilité et la réactivité de la cellulose aux enzymes. Ce prétraitement peut être précédé d'autre(s) traitement(s).

Les brevets US-8057639 et US-8512512 proposent un procédé comprenant une première étape d'hydrolyse de l'hémicellulose en sucres C5 dans des conditions douces les préservant ainsi de leur dégradation. Cette étape est réalisée dans un premier réacteur sous une pression de 1.5 bar ou plus par injection de vapeur, à une température de 110°C ou plus, et éventuellement en présence d'acide faible. Après cette étape, un lavage est réalisé pour extraire et récupérer les jus de sucres C5 avant d'envoyer la biomasse restante, enrichie en cellulose et lignine, dans une seconde étape (second réacteur) où a lieu l'explosion à la vapeur. Ce second réacteur opère à une pression plus élevée que le premier réacteur avec une injection de vapeur à pression élevée qui provoque une détente brutale de la biomasse (explosion à la vapeur).

La demande de brevet US-2012/0104313 propose aussi une étape de traitement préalable à une explosion à la vapeur par contact de la biomasse avec de l'eau ou de la vapeur à 100-210°C pendant 1mn-24h. Après séparation de la phase liquide enrichie en hémicellulose, le solide est ensuite transféré à l'étape d'explosion à la vapeur.

Le brevet EP-2610346 décrit un procédé de traitement de la biomasse lignocellulosique en 4 étapes : un traitement dans un liquide à une température de 100-150°C, suivie d'une séparation du liquide/solide, suivie d'un prétraitement de la partie solide obtenue à 100-210°C en présence d'eau ou de vapeur pendant 1mn-24h et se terminant par une séparation liquide/solide.

Dans le cadre de ses recherches, la demanderesse a mis en évidence qu'il était possible d'améliorer encore significativement les performances des procédés connus de prétraitement de la biomasse. En effet, la présence de cendres et de groupements acétyls à des niveaux variables dans la biomasse est susceptible d'en modifier les propriétés. Néanmoins, les mesures du taux de cendres et le dosage des groupements acétyls sont coûteux en termes de temps et difficiles à mettre en œuvre pour permettre le contrôle en continu d'un procédé.

La demanderesse a ainsi trouvé qu'en ajustant la composition de la liqueur d'imprégnation en fonction du pouvoir acide des flux de liquides issus du procédé de prétraitement de la biomasse (liqueur séparée après imprégnation et/ou l'un quelconque des flux de liquides soutirés au cours du prétraitement de la biomasse), il était possible d'améliorer significativement les performances et la stabilité du procédé de traitement de la biomasse. Il est en effet ainsi possible, en ajustant en temps réel la composition de la liqueur d'imprégnation mise en œuvre, de contrecarrer la variabilité intrinsèque de la biomasse et assurer la stabilité des performances avales, en particulier en termes de réactivité de la biomasse traitée sur les cellulases.

### Résumé de l'invention

Ainsi, la présente invention propose un procédé de traitement continu d'une biomasse lignocellulosique pour la production de jus sucrés, comprenant les étapes suivantes :
a) l'imprégnation de la biomasse au moyen d'une liqueur d'imprégnation présentant un pH compris entre 0,1 et 7, par mise en contact de ladite biomasse avec ladite liqueur d'imprégnation,
b) la séparation de la biomasse imprégnée obtenue à l'étape a) de manière à produire une biomasse humide ayant une teneur en matière sèche d'au moins 15% en poids, et une liqueur séparée,
c) le prétraitement de la biomasse humide obtenue à l'étape b) pour produire une biomasse prétraitée, au cours duquel un ou plusieurs flux de liquides est soutiré,
   caractérisé en ce qu'il comprend
d) l'analyse de la composition de la liqueur séparée issu de l'étape b) et/ou de l'un ou plus des flux de liquides soutirés au cours du prétraitement de la biomasse, et
e) l'ajustement de la composition de la liqueur d'imprégnation mise en œuvre à l'étape a) de manière à maintenir constant le pouvoir acide de ladite liqueur séparée issu de l'étape b) et/ou desdits flux de liquides soutirés au cours du prétraitement de la biomasse, tout au long du procédé.

On entend par jus sucrés, au sens de la présente demande, tout mélange aqueux de sucres issus de la lignocellulose, par exemple du glucose, du xylose, du mannose, du galactose et de l'arabinose.

Il est en effet du mérite de la demanderesse d'avoir mis en évidence qu'il était possible de compenser la variation intrinsèque de composition de la biomasse pour garantir que la biomasse traitée présente toujours sensiblement le même niveau de réactivité vis-à-vis des cellulases. La demanderesse a en effet découvert qu'en contrôlant le pouvoir acide de certains flux de liquides générés dans le procédé, et en ajustant la composition de la liqueur acide d'imprégnation de manière à maintenir constant ce pouvoir acide, il était possible de réguler les procédés de traitements de la biomasse, malgré les variations intrinsèques de composition de celle-ci.

Dans le cadre de la présente invention, l'acronyme "MS" désigne la teneur en matière sèche de la biomasse, qui peut en particulier être mesurée selon la norme ASTM E1756 - 08(2015) « Standard Test Method for Détermination of Total Solids in Biomass".

### Description détaillée

Le procédé selon l'invention est un procédé continu de traitement d'une biomasse lignocellulosique avant hydrolyse enzymatique.

Il s'insère dans les procédés visant à produire des sucres de deuxième génération à partir desquels de nombreuses voies biochimiques permettent d'obtenir des molécules oxygénées (par exemple des alcools comme éthanol, butanol...).

Ce procédé est compatible avec les procédés de production de sucres 2G (c'est-à-dire obtenus à partir de biomasse lignocellulosique) ou plus largement de molécules bio-sourcées (c'est-à-dire à partir de substrats naturels ou dérivés de substrats naturels).

### La biomasse

Le procédé selon l'invention propose de traiter de manière continue une biomasse lignocellulosique.

Cette biomasse peut provenir de sources variées, comme des substrats ligneux comme différents bois (feuillus et résineux), des coproduits issus de l'agriculture (pailles de blé, rafles de maïs, etc...) ou d'autres industries agroalimentaires, papetières, etc...

Selon les biomasses utilisées (pailles, bois, etc...), une étape de broyage, préalable à sa mise en œuvre dans le procédé de l'invention, peut être envisagée pour obtenir une granulométrie compatible avec les moyens technologiques et les conditions opératoires des étapes du procédé. Pour cela, un simple déchiquetage peut être suffisant, mais un broyage avec ou sans affinage peut être requis.

De façon générale, la biomasse mise en œuvre à l'étape a) du procédé de l'invention présente une taille de particule (la plus grande taille) d'au plus 300mm. Le plus souvent, le broyage des pailles se fait avec des grilles de 5 à 100 mm et le bois est déchiqueté en plaquettes parallélépipédiques avec une longueur comprise entre 20 et 160 mm, une largeur comprise entre 10 et 100 mm et une épaisseur comprise entre 2 et 20 mm.

La biomasse est de préférence amenée à l'étape d'imprégnation via une première zone de transfert. Selon un mode particulier de réalisation, la zone de transfert et la zone d'imprégnation peuvent être séparées par un bouchon de biomasse qui empêche la remontée de liquide de ladite première zone vers la zone de transfert ou encore plus en amont.

### Etape de préparation de la liqueur d'imprégnation

La liqueur d'imprégnation mise en œuvre dans le procédé selon l'invention est de préférence une liqueur acide, comprenant au moins un acide, et optionnellement de l'eau.

En effet, certaines biomasses présentent une teneur en matière sèche très faible (par exemple inférieure à 50% MS) et donc une teneur en eau suffisamment élevée pour qu'il ne soit pas nécessaire d'ajouter encore de l'eau pour opérer l'imprégnation. Dans ce cas, la liqueur d'imprégnation est préparée en introduisant uniquement de l'acide qui se mélangera, au cours de l'étape d'imprégnation, à l'eau déjà présente dans la biomasse.

En particulier, la liqueur acide présente un pH compris entre 0,1 et 7,0, de préférence entre 0,1 et 6, et préférentiellement entre 0,1 à 2.

Selon un mode préféré de réalisation, la liqueur comprend au moins un acide et de l'eau.

De préférence, la liqueur acide est une solution aqueuse d'acide fort, par exemple choisi parmi l'acide sulfurique, l'acide chlorhydrique, l'acide nitrique à une teneur en acide comprise entre 0,5 et 8% en poids d'acide, par rapport au poids total de la liqueur. Selon un mode préféré de réalisation, la liqueur d'imprégnation est une solution d'acide sulfurique.

Ce type de liqueur est bien connu de l'homme du métier et tout acide habituellement utilisé pour l'imprégnation convient. La quantité d'acide et la température de la liqueur sont généralement fixées. Les moyens pour obtenir et maintenir la température sont connus de l'homme du métier.

Le procédé selon l'invention peut mettre en œuvre une étape de préparation de la liqueur d'imprégnation préalable à l'étape a) d'imprégnation.

Dans cette étape, la liqueur d'imprégnation est de préférence chauffée à une température allant de 10 à 95°C. Cette étape peut être réalisée dans une zone de préparation de liqueur, située en amont de la zone d'imprégnation.

On peut employer des appareils variés comme, par exemple, une cuve de mélange munie d'un système d'agitation ou un mélangeur (de préférence un mélangeur statique). De préférence, l'appareil est équipé de capteurs de mesure de pH et de débit pour l'eau, l'acide, la liqueur recyclée. L'ensemble de ces capteurs permet de mettre en place une régulation qui équilibre les débits et les acidités de manière à avoir une marche continue stable aux conditions souhaitées.

L'appareil de préparation de la liqueur peut être équipé pour permettre le chauffage de leur contenu au moyen par exemple, d'une double enveloppe, de serpentins et/ou d'échangeurs disposés sur la boucle de recirculation (décrite ci-dessous) à côté ou directement sur lesdits appareils (cuve, mélangeur...). L'appareil utilisé pour la préparation de la liqueur peut notamment être relié à l'imprégnateur par une ou plusieurs conduites transportant la liqueur.

La liqueur peut ainsi être préparée avec la concentration et le débit adéquats qui permettent d'obtenir le pouvoir acide souhaité pour les flux de liquides soutirés en aval de l'imprégnation.

La préparation de liqueur est aussi une étape qui permet de réguler ses paramètres opératoires comme, par exemple, la température, le pH ou toute autre caractéristique. La concentration acide adéquate est réglée grâce à des appoints en acide et/ou en eau.

### Etape d'imprégnation

Le procédé selon l'invention met en œuvre une étape a) d'imprégnation de la biomasse au moyen d'une liqueur d'imprégnation présentant un pH compris entre 0,1 et 7, par mise en contact de ladite biomasse avec ladite liqueur d'imprégnation.

De préférence, dans le procédé selon l'invention, l'étape d'imprégnation est opérée à une température allant de 10 à 95°C, et le temps de séjour de la biomasse dans ladite étape d'imprégnation est compris entre 20 secondes et 12 heures. De manière préférée, le temps de séjour de la biomasse est compris entre 30 secondes et 60 minutes. L'étape d'imprégnation est de manière préférée réalisée à pression atmosphérique.

L'étape d'imprégnation est réalisée par mise en contact de la biomasse avec la liqueur d'imprégnation. Ce contactage peut être opéré par exemple par trempage ou par aspersion. Au cours de l'imprégnation, il est généralement préférable de maintenir le niveau de liqueur quasi constant par apport de liqueur d'imprégnation.

L'étape d'imprégnation peut être opérée en batch ou en continu. De manière préférée, cette étape est opérée en continu. L'imprégnation peut être effectuée dans un ou plusieurs réacteurs d'imprégnation, de préférence dans un seul réacteur d'imprégnation.

L'étape d'imprégnation est réalisée dans des équipements connus de l'homme du métier, par exemple dans un réacteur agité, par traversée horizontale ou verticale de la biomasse dans un lit de liqueur, par aspersion sur un tapis transportant la biomasse ou dans une vis de transport.

Le réacteur d'imprégnation (ou imprégnateur) est généralement muni d'un ou plusieurs outils qui transfère(nt) la biomasse lignocellulosique depuis son entrée vers l'ouverture de sortie. Ces outils peuvent être par exemple des vis ou des tapis. L'imprégnateur est par ailleurs équipé d'une ou plusieurs conduites pour amener l'acide, l'eau ou la liqueur acide ainsi que, de manière optionnelle, d'une ou plusieurs conduites pour soutirer de la liqueur acide.

Selon un mode préféré de réalisation, la biomasse imprégnée de liqueur peut être égouttée au cours de l'imprégnation afin d'extraire au moins une partie de la liqueur acide, avant d'être envoyée à l'étape b) de séparation. Dans ce mode de réalisation, la teneur en matière sèche de la biomasse égoutée est comprise entre 10% et 40% en poids, et de préférence entre 15 et 30% en poids.

Le réacteur d'imprégnation (imprégnateur) peut être équipé d'une ou plusieurs conduites pour amener de la liqueur d'imprégnation lorsqu'elle est préparée dans une étape préalable, ainsi que d'une ou plusieurs conduites d'entrée d'eau et/ou d'acide, et de sortie de liqueur.

Selon un mode très particulier de réalisation, l'étape d'imprégnation peut être mise en œuvre dans un réacteur d'imprégnation (ou imprégnateur) de forme tubulaire et vertical ou incliné avec un angle inférieur à 60° par rapport à la verticale. Ce réacteur comporte notamment 2 zones d'imprégnation superposées et de préférence situées sur le même axe. La zone du bas est appelée première zone d'imprégnation et reçoit par une ouverture la biomasse pressée issue de la première zone de transfert. La zone située au-dessus (zone du haut) est appelée seconde zone d'imprégnation, elle reçoit la biomasse humide égouttée provenant de la première zone d'imprégnation. Dans ce mode de réalisation, le réacteur d'imprégnation (imprégnateur) peut être muni d'une ou plusieurs vis qui transfère(nt) la biomasse par le bas de la première zone d'imprégnation vers l'ouverture de sortie de par le haut de la seconde zone d'imprégnation. La première zone d'imprégnation (donc la zone où a lieu l'imprégnation de la biomasse par la liqueur d'imprégnation) correspond à l'espace rempli par la liqueur d'imprégnation. La seconde zone d'imprégnation ne contient en particulier pas de phase liquide continue. Il est particulièrement avantageux de maintenir une répartition constante entre la première zone d'imprégnation et la seconde zone d'imprégnation. Pour ce faire, le réacteur est équipé d'un système de détection (capteur de niveau), avec de préférence un système de régulation du niveau de liqueur, permettant de garantir un remplissage au niveau souhaité. L'effet de compression de la biomasse lors de la formation du bouchon (au niveau de la vis de transfert amenant la biomasse dans vers l'étape d'imprégnation) et de décompression en entrée de la première zone d'imprégnation remplie de liqueur permet de mieux gorger la biomasse (effet d'éponge). La biomasse est transférée à travers la première zone où elle est imprégnée vers la seconde zone d'imprégnation située au-dessus du niveau de la liqueur. Dans la seconde zone d'imprégnation, une partie de la liqueur imprégnée se sépare de la biomasse imprégnée par égouttage durant l'ascension dans la seconde zone d'imprégnation, la liqueur égouttée (appelée égouttât) étant extraite de la seconde zone d'imprégnation pour être recyclée. De préférence, la seconde zone d'imprégnation est munie de grille(s) retenant la biomasse humide dans la seconde zone d'imprégnation, grille qui laisse donc s'écouler la liqueur égouttée de la seconde zone d'imprégnation dans la première zone d'imprégnation.

### Etape de séparation de la biomasse imprégnée

A l'issue de l'étape a) d'imprégnation, le procédé selon l'invention met en œuvre une étape b) de séparation de la biomasse imprégnée obtenue à l'étape a) de manière à produire une biomasse humide ayant une teneur en matière sèche d'au moins 15% en poids, et une liqueur séparée.

L'étape de séparation peut être effectuée par toute technique connue de l'homme du métier, par exemple par égouttage, décantation, centrifugation ou pressage de la biomasse imprégnée, ou l'association de ces techniques. Selon un mode préféré de réalisation, la séparation est réalisée par pressage.

En particulier, l'étape b) de séparation peut comprendre l'égouttage de la biomasse humide pour atteindre une teneur en matière sèche comprise entre 10% et 40% en poids, puis un seconde séparation, par exemple par pressage, pour atteindre une teneur en matière sèche supérieure comprise entre 40 et 70%. Ce mode de réalisation est particulièrement préféré lorsqu'aucun égouttage n'a été conduit lors de l'étape a) d'imprégnation.

De préférence, à l'issue de l'étape b) de séparation, la biomasse humide présente une teneur en matière sèche comprise entre 25% et 70% en poids, et de préférence entre 40 et 65% en poids.

Dans un mode particulier de réalisation de l'invention, on peut réaliser un pressage de la biomasse humide concomitant à son transfert vers l'étape c) de prétraitement, notamment lorsque cette dernière met en œuvre le procédé de cuisson qui est décrite ci-après. Cette mise en œuvre de l'étape b) de séparation est par exemple assurée par une vis appelée « plug screw feeder », connue de l'homme du métier. Cette vis présente une partie de forme conique, ladite partie conique étant reliée à l'entrée du réacteur de prétraitement. Un bouchon de biomasse se crée au bout de cette partie conique juste avant l'entrée dans le réacteur de prétraitement. La formation d'un bouchon de biomasse lignocellulosique pressée assure l'étanchéité à la pression du réacteur de cuisson susceptible d'être mis en œuvre à l'étape c) de prétraitement. La vis de transfert peut également être munie d'une ou plusieurs conduites de soutirage de la liqueur usée (dite pressât) séparée lors du pressage.

Une extraction importante de la liqueur d'imprégnation usée au cours du présent procédé permet d'augmenter sensiblement le taux de matière sèche de la biomasse pour son traitement ultérieur et sa transformation en sucres. L'obtention d'une biomasse présentant une teneur en matière sèche élevée peut permettre des économies d'énergie pour chauffer le milieu à la température de prétraitement souhaitée, et d'obtenir une biomasse prétraitée ayant également une teneur en MS élevée. Le convoyage de la biomasse prétraitée est ainsi facilité vers l'étape suivante d'hydrolyse enzymatique, et l'hydrolyse enzymatique d'une biomasse à haute teneur en MS permet d'obtenir une solution de sucres (hydrolysés) concentrée, permettant de viser des titres (teneur en alcools) élevés en fermentation, et donc de réduire les coûts liés aux étapes de séparation, notamment par distillation, pour valoriser ces alcools. Par ailleurs, opérer le procédé en maintenant une teneur en matière sèche de biomasse élevée permet de diminuer le volume des cuves de fermentation (et d'hydrolyse) et de réduire la quantité d'eaux usées générées par le procédé.

La zone de séparation mise en œuvre à l'étape b) peut notamment être munie d'une conduite de soutirage de la liqueur usée résultante (appelée pressât) séparée de la biomasse humide.

### Etape de prétraitement

Le procédé selon l'invention met également en œuvre une étape c) de prétraitement de la biomasse humide obtenue à l'étape b) pour produire une biomasse prétraitée, au cours duquel un ou plusieurs flux de liquides est soutiré

Le prétraitement vise notamment à modifier les propriétés physiques et physicochimiques de la fraction cellulosique, telles que son degré de polymérisation et son état de cristallinité. En particulier, le prétraitement comprend une hydrolyse de l'hémicellulose présente dans la biomasse et permet une meilleure accessibilité de la cellulose aux enzymes.

Selon un mode préféré de réalisation, l'étape c) de prétraitement de la biomasse humide est réalisée par cuisson, de préférence par explosion à la vapeur.

Cette cuisson s'effectue par exemple dans une zone de cuisson opérée à une température comprise entre 100°C et 250°C, et plus préférentiellement entre 130°C et 230°C, à une pression comprise entre 0,1 et 4 MPa. Le temps de séjour de la biomasse dans la zone de cuisson est compris entre 10 secondes et 4 heures, et plus préférentiellement entre 3 minutes et 1 heure.

La cuisson peut avoir lieu en batch ou en continu. Elle peut être réalisée dans tout équipement connu de l'homme du métier, par exemple un réacteur agité, un réacteur tubulaire horizontal muni d'une vis de transport, un réacteur batch non agité, etc... L'énergie thermique nécessaire à la cuisson peut être apportée via un échange thermique avec un fluide caloporteur (indirect), par chauffage électrique, ou bien par injection directe d'un fluide chaud, par exemple de l'eau pressurisée ou de la vapeur.

La sortie de la biomasse prétraitée en fin de cuisson peut se faire par décompression rapide, par décompression lente, après une baisse de température induite par un échange thermique direct ou indirect, etc...

Dans un mode de réalisation préféré, la zone de cuisson est chauffée à la vapeur par injection directe et est suivie d'une décompression brutale du milieu, ce procédé est connu sous le nom d'explosion à la vapeur ("SteamEx" ou "Steam Explosion" selon la terminologie anglo-saxonne). C'est un procédé dans lequel la biomasse lignocellulosique est portée rapidement à haute température par injection de vapeur sous pression. L'arrêt du traitement s'effectue par décompression brutale.

Les conditions opératoires du procédé d'explosion à la vapeur peuvent notamment être les suivantes :
- la vapeur est injectée directement dans le réacteur ;
- la température du réacteur est généralement comprise entre 150 et 250°C, de préférence comprise entre 160°C et 220°C,
- la pression est comprise entre 0,5 et 2,5 MPa, plus préférentiellement comprise entre 0,8 et 2,0 MPa.
- le temps de séjour avant la phase de détente varie de 10 secondes à 25 minutes, et de préférence entre 3 minutes et 15 minutes.

L'explosion à la vapeur peut être réalisée en batch ou en continu et l'étape de dépressurisation qui permet de déstructurer la biomasse peut se dérouler en une ou plusieurs étapes.

Au cours de l'étape de prétraitement, différents flux de liquides peuvent être soutirés. Il s'agit notamment des fluides chauds (liquide ou vapeur) injectés pour assurer le prétraitement, tels que l'eau pressurisée ou la vapeur d'eau.

La vapeur récupérée est avantageusement recyclée après compression à l'étape d'explosion à la vapeur, ou éventuellement est recyclée sur les utilités du site.

Selon un mode particulier de réalisation, l'étape de prétraitement peut être conduite dans un réacteur tubulaire horizontal (i.e. qui peut être très légèrement incliné pour l'écoulement du liquide) par exemple muni d'une vis de transfert de la biomasse à travers les zones successives. La vis assure le transport de la biomasse de façon continue, la vitesse de la vis étant réglée pour remplir les conditions de temps de séjour. Au bout de la vis (au bout du réacteur), la biomasse est entraînée très rapidement par la vapeur vers une zone de détente dans une ligne appelée « blowline » qui a un diamètre réduit par rapport à la zone de cuisson. La zone de détente comprend une ligne dans laquelle la biomasse circule et passe dans un organe de restriction de section puis, après avoir franchi la restriction, subit une détente brutale. La « blowline » possède un organe de restriction de section qui peut être un orifice ou une vanne à ouverture réglable (vanne à diaphragme par exemple) permettant une section de passage faible. Au niveau de cette restriction, la biomasse arrive avec une vitesse de transport très élevée, et elle subit une variation de pression rapide et importante, puis une détente brutale après avoir franchi la restriction, ce qui déstructure la biomasse cuite. C'est pour cela que l'on parle d'explosion à la vapeur. Une fois la zone de détente passée, la biomasse est entraînée par la vapeur dans la suite de la « blowline » qui possède un diamètre plus grand que la restriction (ou qui retrouve son diamètre en amont de la restriction) et qui amène la biomasse jusqu'à une zone de séparation de la vapeur, par exemple par un cyclone.

La biomasse prétraitée issue de l'étape c) présente maintenant une accessibilité de la cellulose aux enzymes suffisante pour être traitée par hydrolyse enzymatique pour la production de sucres 2G.

### Analyse de l'acidité des flux et ajustement de la composition de la ligueur d'imprégnation

Le procédé selon l'invention est caractérisé en ce qu'il met en œuvre une étape d) d'analyse de la composition de la liqueur séparée issu de l'étape b) et/ou de l'un ou plus des flux de liquides soutirés au cours du prétraitement de la biomasse.

En particulier, les flux de liquides soutirés au cours du prétraitement de la biomasse sont les flux de liquides soutirés en aval de l'étape a) d'imprégnation, et en amont des étapes ultérieures de traitement de la biomasse, en particulier en amont de l'étape de cuisson.

Selon un mode préféré de réalisation, l'étape d) met en œuvre l'analyse la composition de la liqueur séparée issu de l'étape b) (appelée pressât).

En effet, il est du mérite de la demanderesse d'avoir mis en évidence que le contrôle de la composition, et en particulier du pouvoir acide de la liqueur séparée issu de l'étape b) et/ou de l'un ou plus des flux de liquides soutirés au cours du prétraitement de la biomasse pouvait permettre de contrebalancer la variabilité intrinsèque de la biomasse et d'assurer la stabilité des performances avales, en particulier en termes de réactivité de la biomasse traitée sur les cellulases.

En particulier, l'analyse de la composition de la liqueur séparée issu de l'étape b) et/ou de l'un ou plus des flux de liquides soutirés au cours du prétraitement comprend la mesure du pouvoir acide de ladite liqueur séparée issu de l'étape b) et/ou desdits flux de liquides soutirés au cours du prétraitement de la biomasse, tout au long du procédé, de préférence par mesure du pH de ladite liqueur et/ou desdits flux de liquides, les mesures étant notamment réalisées en continu ou à une fréquence donnée.

Le pouvoir acide se définit comme la quantité d'ions H⁺ présents dans le liquide considéré. Il peut être mesuré par différents paramètres, par des méthodes connues de l'homme du métier. Le pouvoir acide peut par exemple être mesuré par un dosage des acides contenus dans la solution par titrimétrie (suivi de l'évolution du pH au cours d'un ajout cadencé d'une solution base), par mesure du pH de la solution et/ou par mesure de la conductivité de ladite solution. Idéalement, lorsque les équipements permettant la mesure du pH sont suffisamment précis, une mesure du pH des liquides est préférée pour caractériser le pouvoir acide.

Lorsque les niveaux de pH sont très faibles et que la mesure de pH risque de ne pas donner des résultats suffisamment précis, celle-ci peut, de préférence, s'accompagner d'une mesure de la conductivité.

Le pH de la liqueur séparée issu de l'étape b) et/ou de l'un ou plus des flux de liquides soutirés au cours du prétraitement est de préférence compris entre 0,1 et 7, de préférence entre 0,5 et 4, et de préférence encore entre 0,7 et 2,5.

La conductivité de la liqueur séparée issu de l'étape b) et/ou de l'un ou plus des flux de liquides soutirés au cours du prétraitement peut quant à elle être comprise entre 5 et 250mS/cm et plus préférentiellement entre 15 et 150mS/cm, lorsqu'elle est mesurée à 70°C.

Le procédé selon l'invention met en œuvre une régulation de la composition de la liqueur d'imprégnation utilisée à l'étape a) afin de maintenir constant le pouvoir acide d'un au moins des flux de liquides soutirés au cours du procédé de l'invention.

En particulier, le procédé selon l'invention met en œuvre une étape e) d'ajustement de la composition de la liqueur d'imprégnation mise en œuvre à l'étape a) de manière à maintenir constant le pouvoir acide de ladite liqueur séparée issu de l'étape b) et/ou desdits flux de liquides soutirés au cours du prétraitement de la biomasse, tout au long du procédé.

On entend, au sens de la présente demande, que le pouvoir acide est « maintenu constant » lorsque la grandeur qui le caractérise (pH, conductivité, dosage des acides...) ne varie généralement que de plus ou moins 10 à 20% pendant toute la durée de fonctionnement du procédé. Ainsi, en particulier, la composition de la liqueur d'imprégnation devrait être ajustée de manière à garantir que le pH de la liqueur séparée issue de l'étape b) et/ou desdits flux de liquides soutirés au cours du prétraitement de la biomasse ne varie pas de plus de 20% autour de sa valeur initiale, notamment autour de sa valeur de consigne, de préférence de plus ou moins 10% autour de sa valeur initiale (donc, notamment, à +/-20% près, de préférence à +/- 10% près par rapport à la valeur de pouvoir acide visée). Pour les valeurs de pH très basses, et typiquement inférieures à 1.5, on considère la stabilité du pH comme une variation de plus ou moins 0.1 unité pH autour de la valeur de consigne. Selon un mode particulier de réalisation, lorsque le pouvoir acide est caractérisé par la conductivité, la composition de la liqueur d'imprégnation devrait être ajustée de manière à garantir que la conductivité de la liqueur séparée issue de l'étape b) et/ou desdits flux de liquides soutirés au cours du prétraitement de la biomasse ne varie pas de plus de 20% autour de sa valeur initiale, et de préférence plus de 10% autour de sa valeur initiale.

Ainsi, lorsque le pouvoir acide du flux considéré diminue au cours du fonctionnement du procédé selon l'invention, on augmente l'acidité (par abaissement du pH) de la liqueur d'imprégnation introduite à l'étape a), et inversement, lorsque le pouvoir acide du flux considéré augmente au cours du fonctionnement du procédé selon l'invention, on diminue l'acidité (par dilution) de la liqueur d'imprégnation introduite à l'étape a).

En particulier, l'ajustement est réalisé par ajout (ou appoint) d'eau et/ou d'acide dans ladite liqueur d'imprégnation mise en œuvre à l'étape a).

Les appoints d'eau ou d'acide peuvent être effectués soit directement à l'étape a) d'imprégnation de la biomasse, soit lors de préparation de la liqueur d'imprégnation. Il peut se faire directement en ligne ou dans un équipement (mélangeur, cuve, ou autre).

### Etape de recyclage

Selon un mode particulier de réalisation, le procédé selon l'invention met en œuvre une étape de recyclage de la liqueur séparée issu de l'étape b), des flux de liquides soutirés au cours du prétraitement de la biomasse et/ou des flux de liquides soutirés au cours des étapes ultérieures de traitement de la biomasse, par exemple par hydrolyse enzymatique et/ou fermentation alcoolique, vers l'étape a) d'imprégnation.

Dans le cas où un recyclage est mis en œuvre, l'ajustement de la composition de la liqueur d'imprégnation est d'autant plus important que les flux recyclés présentent un pouvoir acide variable.

En effet, la demanderesse a mis en évidence que l'imprégnation générait des liqueurs usées (pressât et égouttât) appauvries en acide et des flux de liquides soutirés au cours du prétraitement de la biomasse présentant une acidité résiduelle. Sans vouloir être lié par une théorie, il est possible que ces variations d'acidité puissent être dues à une basicité de la biomasse qui neutralise une partie de l'acide de la liqueur d'imprégnation introduite dans le procédé de l'invention. De manière surprenante, ces flux, notamment la liqueur usée, présentent également un effet tampon qui peut être dû aux extractibles de la biomasse. Ainsi, leur recyclage modifie significativement les propriétés de la liqueur d'imprégnation. Par la mise en œuvre des appoints d'eau et d'acide décrits dans la présente demande, la demanderesse a ainsi observé qu'il était possible de compenser les variations de qualité de la liqueur d'imprégnation liées aux recyclages afin de maintenir constant les bonnes propriétés de réactivité aux cellulases de la biomasse traitée.

Ainsi, lorsqu'un recyclage d'un ou plusieurs flux de liquides du procédé est mis en œuvre, leur composition est analysée de la même manière que celle de la liqueur séparée issu de l'étape b) ou de des flux de liquides soutirés au cours du prétraitement de la biomasse, et la composition de la liqueur d'imprégnation mise en œuvre à l'étape a) est ajustée de manière à maintenir constant le pouvoir acide dudit flux de liquides recyclé, tout au long du procédé.

On prépare ainsi, dans le cadre du présent procédé une liqueur présentant le pH souhaité pour maintenir une bonne imprégnation tout au long du procédé. Une partie de l'appoint d'acide permet de contrer la basicité de la biomasse qui peut être mesurée au préalable. Une seconde partie de cet appoint permet de contrer le caractère tampon des liqueurs usées. Les appoints d'eau permettent d'ajuster le débit de liqueur d'imprégnation introduite dans l'étape a).

Dans certain cas, on peut aussi installer une ou deux purges juste après la séparation de la liqueur réalisée à l'étape b) (pressage ou égouttage) pour empêcher une trop grande concentration de molécules issues de la biomasse.

Il est également possible d'envisager une étape de traitement de la liqueur à recycler pour la purifier et diminuer l'usage de purge.

De même, dans certains cas, il est possible d'utiliser, pour les appoints, différentes sources d'eau provenant du procédé dans lequel s'intègre la chaine d'imprégnation/prétraitement. Par exemple, l'appoint d'eau peut venir du traitement des eaux, des vinasses issues de la première colonne de distillation ou des flegmasses provenant de certaines coupes de la distillation principale, ou une combinaison de ce genre de flux. Ces vinasses et ces flegmasses peuvent éventuellement subir un traitement pour être purifiées.

### Traitement de la biomasse prétraitée par hydrolyse enzymatique et fermentation alcoolique

Le procédé selon l'invention peut en particulier comprendre, après l'étape c) de prétraitement, une étape de traitement d'au moins une partie de la biomasse prétraitée, par hydrolyse enzymatique, pour produire lesdits jus sucrés. Selon un mode préféré de réalisation, une partie au moins des jus sucrés est soumise à une fermentation alcoolique.

Les conditions de l'hydrolyse enzymatique et de la fermentation consécutive ou simultanée sont adaptées aux produits souhaités et sont connues de l'homme du métier.

Le procédé selon l'invention trouve une application particulièrement intéressante dans un procédé de préparation de sucres à partir de biomasse lignocellulosique et dans le procédé de production d'éthanol à partir desdits jus sucrés. De tels procédés sont connus. Un procédé de préparation de sucres à partir de biomasse lignocellulosique comporte un prétraitement, qui est avantageusement une explosion à la vapeur, suivi d'une hydrolyse enzymatique. Le procédé de production d'éthanol à partir des sucres comprend en outre une fermentation alcoolique desdits sucres.

Dans le cadre du présent procédé, les débits de la liqueur d'imprégnation, de l'eau, des liqueurs séparées (pressât ou l'égouttât), sont mesurés par tout équipement connu de l'homme du métier (par exemple, débitmètre, niveau dans une capacité, autre...).

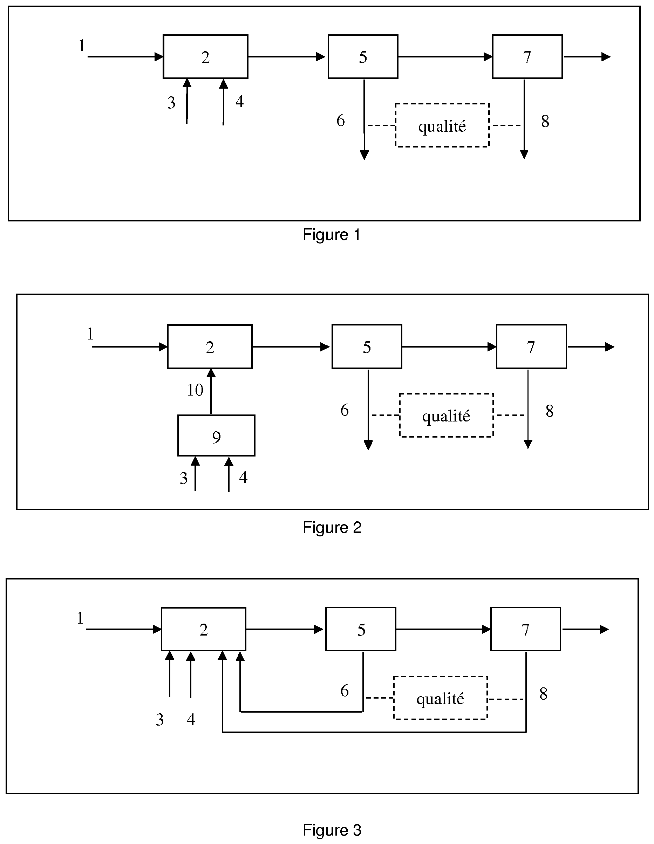

Les figures 1 à 4 de la présente demande illustrent, de manière non limitative, la mise en œuvre du procédé selon l'invention.

La biomasse broyée est introduite par la conduite 1 dans la zone d'imprégnation 2. Cette zone contient la liqueur d'imprégnation, composée d'acide introduit par la conduite 3, et optionnellement d'eau introduite par la conduite 4.

Dans le mode de réalisation illustré à la figure 2, la liqueur d'imprégnation est préparée dans une étape préalable à l'imprégnation, dans une zone de préparation de la liqueur 9, par introduction d'acide (3) et optionnellement d'eau (4). La liqueur ainsi préparée est introduite dans la zone d'imprégnation 2 par la conduite 10.

La biomasse imprégnée obtenue est ensuite transférée vers une zone de séparation 5 de manière à produire une biomasse humide et une liqueur séparée 6.

Selon un mode de réalisation, les caractéristiques de la liqueur séparée 6 sont mesurées au moyen de capteurs (pH, conductivité, débit...) et les quantités d'acide et/ou d'eau introduites par les conduites 3 et 4 sont ajustées de manière à maintenir constant le pouvoir acide de ladite liqueur séparée 6 pendant toute la durée d'opération du procédé.

La biomasse humide obtenue lors de la séparation est ensuite prétraitée dans une zone 7 de prétraitement de laquelle un ou plusieurs flux de liquides 8 est soutiré.

Selon un mode de réalisation particulier, les caractéristiques flux de liquides 8 sont mesurées au moyen de capteurs (pH, conductimétrie, débit...) et les quantités d'acide et/ou d'eau introduites par les conduites 3 et 4 sont ajustées de manière à maintenir constant le pouvoir acide desdits flux de liquides 8 pendant toute la durée d'opération du procédé.

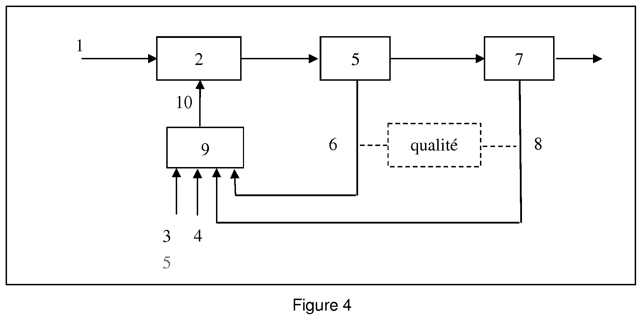

Les figures 3 et 4 illustrent le possible recyclage de la liqueur séparée 6 et/ou des flux de liquides 8 vers la zone d'imprégnation 2.

### Exemples

Exemple 1 :

L'exemple 1 présente le mode opératoire selon l'art antérieur. La biomasse (paille de blé) est mise en contact avec une liqueur acide (eau + acide sulfurique) contrôlée en acidité (1,1%pds). La biomasse ainsi imprégnée est prétraitée par cuisson dans un réacteur d'explosion à la vapeur (190°C, 5 minutes de temps de séjour).

En contrôlant uniquement l'acidité de la liqueur introduite à l'étape d'imprégnation, les variations de qualité de la biomasse induisent des variations dans la qualité du produit prétraité. Le tableau suivant exemplifie ce phénomène, les deux tests ont été conduits sur de la paille de blé dans les mêmes conditions, les résultats mettent en évidence la variabilité intrinsèque de la charge.

| | Paille Test 1 | Paille Test 2 |
|---|---|---|
| Acidité liqueur acide | 1,1%pds | 1,1%pds |
| pH pressât | 1,6 | 1,8 |
| Conductivité pressât (mS/cm) | 12 | 20 |
| Teneur en sucres monomérique et oligomérique dans la biomasse prétraitée | 50,3% | 64,8% |

Pour une acidité à 1,1%pds, la production de sucres monomériques et oligomériques peut varier de 50 à 65%pds après l'étape de prétraitement, ceci en raison de la variabilité de la biomasse traitée. On observe également une variation importante dans la qualité de la liqueur d'imprégnation usée (pressât) de 0,2 unité de pH et 8 mS/cm sur la conductivité. Cette variation de qualité de la liqueur usée est directement responsable de la variabilité sur la teneur en sucres dans le produit fini.

### Exemple 2

L'exemple 2 présente le mode opératoire selon l'invention. La biomasse (paille de blé) utilisée dans l'exemple 1 est mise en contact avec une liqueur acide (eau + acide sulfurique). La biomasse imprégnée est introduite dans le réacteur de prétraitement par l'intermédiaire d'une vis, générant un flux liquide appelé pressât. Dans la mise en œuvre selon l'invention, la teneur en acide de la liqueur d'imprégnation est ajustée de façon à maintenir constant la qualité du pressât (pH ou conductivité).

Le tableau suivant illustre les résultats de ce mode opératoire pour une production de plusieurs jours.

| | Test 3 | variation |
|---|---|---|
| pH pressât | 0,61 | +/- 0,1 |
| Conductivité pressât (mS/cm) | 33,5 | +/- 3,2 |
| Teneur en sucres monomérique et oligomérique dans la biomasse prétraitée | 68,7% | +/- 5% |

La régulation du pressât (pH à +/-0,1 ou conductivité à +/-10%) a permis de limiter la variabilité de la qualité de la biomasse prétraitée à +/- 5 points de rendement en sucres (monomériques et oligomériques). L'utilisation du mode de régulation de la liqueur acide selon l'invention permet donc de garantir une qualité constante du produit fini.

## Revendications

1. Procédé de traitement continu d'une biomasse lignocellulosique pour la production de jus sucrés, comprenant les étapes suivantes :
a) l'imprégnation de la biomasse au moyen d'une liqueur d'imprégnation présentant un pH compris entre 0,1 et 7, par mise en contact de ladite biomasse avec ladite liqueur d'imprégnation,
b) la séparation de la biomasse imprégnée obtenue à l'étape a) de manière à produire une biomasse humide ayant une teneur en matière sèche d'au moins 15% en poids, et une liqueur séparée,
c) le prétraitement de la biomasse humide obtenue à l'étape b) pour produire une biomasse prétraitée, au cours duquel un ou plusieurs flux de liquides est soutiré,
**caractérisé en ce qu'**il comprend
d) l'analyse de la composition de la liqueur séparée issu de l'étape b) et/ou de l'un ou plus des flux de liquides soutirés au cours du prétraitement de la biomasse, et
e) l'ajustement de la composition de la liqueur d'imprégnation mise en œuvre à l'étape a) de manière à maintenir constant le pouvoir acide, correspondant à la quantité d'ions H⁺ présents, de ladite liqueur séparée issu de l'étape b) et/ou desdits flux de liquides soutirés au cours du prétraitement de la biomasse, tout au long du procédé.

2. Procédé selon la revendication précédente, **caractérisé en ce que** la liqueur d'imprégnation comprend au moins un acide, et optionnellement de l'eau.

3. Procédé selon l'une des revendications précédentes, **caractérisé en ce qu'**il comprend une étape de préparation de la liqueur d'imprégnation préalable à l'étape a) d'imprégnation.

4. Procédé selon l'une des revendications précédentes, **caractérisé en ce que** l'étape d'imprégnation est opérée à une température allant de 10 à 95°C, et le temps de séjour de la biomasse dans ladite étape d'imprégnation est compris entre 20 secondes et 12 heures.

5. Procédé selon l'une des revendications précédentes, **caractérisé en ce que** la liqueur d'imprégnation présente un pH compris entre 0,1 et 6, et de préférence entre 0,1 et 2.

6. Procédé selon l'une des revendications précédentes, **caractérisé en ce que** la liqueur d'imprégnation est une solution aqueuse d'acide fort, par exemple choisi parmi l'acide sulfurique, l'acide chlorhydrique, l'acide nitrique à une teneur en acide comprise entre 0,5 et 8% en poids d'acide, par rapport au poids total de la liqueur.

7. Procédé selon l'une des revendications précédentes, **caractérisé en ce que** l'étape de séparation peut être effectuée par égouttage, décantation, centrifugation ou pressage de la biomasse imprégnée.

8. Procédé selon l'une des revendications précédentes, **caractérisé en ce que** la teneur en matière sèche de la biomasse humide obtenue à l'étape b) est comprise entre 25% et 70% en poids, et de préférence entre 40 et 65% en poids.

9. Procédé selon l'une des revendications précédentes, **caractérisé en ce que** l'étape c) de prétraitement de la biomasse humide est réalisée par cuisson, de préférence par explosion à la vapeur.

10. Procédé selon l'une des revendications précédentes, **caractérisé en ce que** l'étape d) d'analyse de la composition de la liqueur séparée issu de l'étape b) et/ou de l'un ou plus des flux de liquides soutirés au cours du prétraitement de la biomasse comprend la mesure du pouvoir acide de ladite liqueur séparée issu de l'étape b) et/ou desdits flux de liquides soutirés au cours du prétraitement de la biomasse, tout au long du procédé.

11. Procédé selon la revendication précédente, **caractérisé en ce que** la mesure du pouvoir acide de la liqueur et/ou des flux de liquides s'effectue par mesure du pH ou par mesure de conductivité, les mesures étant réalisées notamment en continu ou à une fréquence donnée.

12. Procédé selon l'une des revendications précédentes, **caractérisé en ce que** lesdits flux de liquides soutirés au cours du prétraitement de la biomasse sont les flux de liquides soutirés en aval de l'étape a) d'imprégnation, et en amont des étapes ultérieures de traitement de la biomasse.

13. Procédé selon l'une des revendications précédentes, **caractérisé en ce que** l'étape e) d'ajustement de la composition de la liqueur d'imprégnation est réalisée par ajout d'eau et/ou d'acide dans ladite liqueur d'imprégnation mise en œuvre à l'étape a), de manière à maintenir constant, tout au long du procédé, le pouvoir acide de la liqueur séparée issu de l'étape b) et/ou des flux de liquides soutirés au cours du prétraitement de la biomasse, notamment à +/- 20% près, de préférence à +/-10% près.

14. Procédé selon l'une des revendications précédentes, **caractérisé en ce qu'**il comprend une étape de recyclage de la liqueur séparée issu de l'étape b), des flux de liquides soutirés au cours du prétraitement de la biomasse et/ou des flux de liquides soutirés au cours des étapes ultérieures de traitement de la biomasse, par exemple par hydrolyse enzymatique et/ou fermentation alcoolique, vers l'étape a) d'imprégnation.

15. Procédé selon l'une des revendications précédentes, **caractérisé en ce qu'**il comprend, après l'étape c) de prétraitement, une étape de traitement d'au moins une partie de la biomasse prétraitée, par hydrolyse enzymatique, pour produire lesdits jus sucrés.

16. Procédé selon l'une des revendications précédentes, **caractérisé en ce qu'**une partie au moins des jus sucrés est soumise à une fermentation alcoolique.

## Patentansprüche

1. Verfahren zur kontinuierlichen Behandlung einer lignocellulosehaltigen Biomasse zur Erzeugung von Zuckersäften, umfassend die folgenden Schritte:
a) das Imprägnieren der Biomasse mittels einer Imprägnierlösung, die einen pH-Wert zwischen 0,1 und 7 aufweist, durch Inkontaktbringen der Biomasse mit der Imprägnierlösung,
b) das Abtrennen der in Schritt a) erhaltenen imprägnierten Biomasse, so dass eine feuchte Biomasse mit einem Trockenmassegehalt von mindestens 15 Gew.-% und eine abgetrennte Lösung erzeugt werden,
c) die Vorbehandlung der in Schritt b) erhaltenen feuchten Biomasse, um eine vorbehandelte Biomasse zu erzeugen, in deren Verlauf ein oder mehrere Flüssigkeitsströme abgezogen werden, **dadurch gekennzeichnet, dass** es umfasst
d) das Analysieren der Zusammensetzung der aus Schritt b) hervorgegangenen abgetrennten Lösung und/oder eines oder mehrerer der im Verlauf der Vorbehandlung der Biomasse abgezogenen Flüssigkeitsströme und
e) das Einstellen der Zusammensetzung der in Schritt a) eingesetzten Imprägnierlösung derart, dass die Stärke der Säure, entsprechend der Menge vorhandener H⁺-Ionen, der aus Schritt b) hervorgegangen abgetrennten Lösung und/oder der im Verlauf der Vorbehandlung der Biomasse abgezogenen Flüssigkeitsströme während des gesamten Verfahrens konstant gehalten wird.

2. Verfahren nach dem vorhergehenden Anspruch, **dadurch gekennzeichnet, dass** die Imprägnierlösung mindestens eine Säure und optional Wasser umfasst.

3. Verfahren nach einem der vorhergehenden Ansprüche, **dadurch gekennzeichnet, dass** es einen Schritt der Zubereitung der Imprägnierlösung vor dem Schritt a) des Imprägnierens umfasst.

4. Verfahren nach einem der vorhergehenden Ansprüche, **dadurch gekennzeichnet, dass** der Schritt des Imprägnierens bei einer Temperatur von 10 bis 95 °C durchgeführt wird und die Verweilzeit der Biomasse in dem Imprägnierschritt zwischen 20 Sekunden und 12 Stunden beträgt.

5. Verfahren nach einem der vorhergehenden Ansprüche, **dadurch gekennzeichnet, dass** die Imprägnierlösung einen pH-Wert zwischen 0,1 und 6 und bevorzugt zwischen 0,1 und 2 aufweist.

6. Verfahren nach einem der vorhergehenden Ansprüche, **dadurch gekennzeichnet, dass** die Imprägnierlösung eine wässrige Lösung einer starken Säure, die beispielsweise unter Schwefelsäure, Salzsäure, Salpetersäure gewählt ist, mit einem Säuregehalt zwischen 0,5 und 8 Gew.-% Säure bezogen auf das Gesamtgewicht der Lösung ist.

7. Verfahren nach einem der vorhergehenden Ansprüche, **dadurch gekennzeichnet, dass** der Schritt des Abtrennens durch Abtropfen, Dekantieren, Zentrifugieren oder Pressen der imprägnierten Biomasse durchgeführt werden kann.

8. Verfahren nach einem der vorhergehenden Ansprüche, **dadurch gekennzeichnet, dass** der Trockenmassegehalt der in Schritt b) erhaltenen feuchten Biomasse zwischen 25 und 70 Gew.-% und bevorzugt zwischen 40 und 65 Gew.-% beträgt.

9. Verfahren nach einem der vorhergehenden Ansprüche, **dadurch gekennzeichnet, dass** der Schritt c) der Vorbehandlung der feuchten Biomasse durch Kochen, bevorzugt durch Dampfexplosion, ausgeführt wird.

10. Verfahren nach einem der vorhergehenden Ansprüche, **dadurch gekennzeichnet, dass** der Schritt d) des Analysierens der Zusammensetzung der aus Schritt b) hervorgegangenen abgetrennten Lösung und/oder eines oder mehrerer im Verlauf der Vorbehandlung der Biomasse abgezogenen Flüssigkeitsströme das Messen der Stärke der Säure der aus Schritt b) hervorgegangenen abgetrennten Lösung und/oder der im Verlauf der Vorbehandlung der Biomasse abgezogenen Flüssigkeitsströme während des gesamten Verfahrens umfasst.

11. Verfahren nach dem vorhergehenden Anspruch, **dadurch gekennzeichnet, dass** das Messen der Stärke der Säure der Lösung und/oder der Flüssigkeitsströme durch Messen des pH-Werts oder durch Leitfähigkeitsmessung erfolgt, wobei die Messungen insbesondere kontinuierlich oder mit einer gegebenen Häufigkeit ausgeführt werden.

12. Verfahren nach einem der vorhergehenden Ansprüche, **dadurch gekennzeichnet, dass** die im Verlauf der Vorbehandlung der Biomasse abgezogenen Flüssigkeitsströme die Flüssigkeitsströme sind, die nach dem Schritt a) des Imprägnierens und vor den späteren Schritten zur Behandlung der Biomasse abgezogen werden.

13. Verfahren nach einem der vorhergehenden Ansprüche, **dadurch gekennzeichnet, dass** der Schritt e) des Einstellens der Zusammensetzung der Imprägnierlösung durch Zugabe von Wasser und/oder von Säure zu der in Schritt a) eingesetzten Imprägnierlösung ausgeführt wird, so dass die Stärke der Säure der aus Schritt b) hervorgegangenen abgetrennten Lösung und/oder der im Verlauf der Vorbehandlung der Biomasse abgezogenen Flüssigkeitsströme während des gesamten Verfahrens konstant gehalten wird, insbesondere bis auf +/-20 %, bevorzugt genau +/- 10 %.

14. Verfahren nach einem der vorhergehenden Ansprüche, **dadurch gekennzeichnet, dass** es einen Schritt der Rückführung der aus Schritt b) hervorgegangenen abgetrennten Lösung, der im Verlauf der Vorbehandlung der Biomasse abgezogenen Flüssigkeitsströme und/oder der im Verlauf der späteren Behandlungsschritte der Biomasse, beispielsweise durch enzymatische Hydrolyse und/oder alkoholische Fermentation, zum Schritt a) des Imprägnierens umfasst.

15. Verfahren nach einem der vorhergehenden Ansprüche, **dadurch gekennzeichnet, dass** es nach dem Schritt c) der Vorbehandlung einen Schritt der Behandlung mindestens eines Teils der vorbehandelten Biomasse durch enzymatische Hydrolyse umfasst, um die Zuckersäfte zu erzeugen.

16. Verfahren nach einem der vorhergehenden Ansprüche, **dadurch gekennzeichnet, dass** mindestens ein Teil der Zuckersäfte einer alkoholischen Fermentation unterzogen wird.

## Claims

1. A process for the continuous treatment of a lignocellulosic biomass for the production of sugary liquors, comprising the following steps:
a) impregnating the biomass using an impregnation liquor with a pH of between 0.1 and 7, by placing said biomass in contact with said impregnation liquor,
b) separating the impregnated biomass obtained in step a) so as to produce a wet biomass with a solids content of at least 15% by weight, and a separated liquor,
c) pretreating the wet biomass obtained in step b) to produce a pretreated biomass, in the course of which one or more liquid streams are withdrawn,
**characterized in that** it comprises
d) analysis of the composition of the liquor separated out obtained from step b) and/or of one or more of the liquid streams withdrawn during the biomass pretreatment, and
e) adjustment of the composition of the impregnation liquor used in step a) so as to keep constant the acidic power, corresponding to the amount of H⁺ ions present, of said liquor separated out obtained from step b) and/or of said liquid streams withdrawn during the biomass pretreatment, throughout the process.

2. The process as claimed in the preceding claim, **characterized in that** the impregnation liquor comprises at least one acid, and optionally water.

3. The process as claimed in either of the preceding claims, **characterized in that** it comprises a step of preparing the impregnation liquor prior to the impregnation step a).

4. The process as claimed in one of the preceding claims, **characterized in that** the impregnation step is performed at a temperature ranging from 10 to 95°C, and the residence time of the biomass in said impregnation step is between 20 seconds and 12 hours.

5. The process as claimed in one of the preceding claims, **characterized in that** the impregnation liquor has a pH of between 0.1 and 6 and preferably between 0.1 and 2.

6. The process as claimed in one of the preceding claims, **characterized in that** the impregnation liquor is an aqueous solution of a strong acid, chosen, for example, from sulfuric acid, hydrochloric acid and nitric acid at an acid content of between 0.5% and 8% by weight of acid relative to the total weight of the liquor.

7. The process as claimed in one of the preceding claims, **characterized in that** the separation step may be performed by draining, decantation, centrifugation or pressing of the impregnated biomass.

8. The process as claimed in one of the preceding claims, **characterized in that** the solids content of the wet biomass obtained in step b) is between 25% and 70% by weight and preferably between 40% and 65% by weight.

9. The process as claimed in one of the preceding claims, **characterized in that** step c) of pretreating the wet biomass is performed by cooking, preferably by steam explosion.

10. The process as claimed in one of the preceding claims, **characterized in that** step d) of analysis of the composition of the liquor separated out obtained from step b) and/or of one or more of the liquid streams withdrawn during the biomass pretreatment comprises measurement of the acidic power of said liquor separated out obtained from step b) and/or of said liquid streams withdrawn during the biomass pretreatment, throughout the process.

11. The process as claimed in the preceding claim, **characterized in that** the measurement of the acidic power of the liquor and/or of the liquid streams is performed by measuring the pH or by measuring the conductivity, the measurements being performed notably continuously or at a given frequency.

12. The process as claimed in one of the preceding claims, **characterized in that** said liquid streams withdrawn during the biomass pretreatment are liquid streams withdrawn downstream of the impregnation step a) and upstream of the subsequent biomass treatment steps.

13. The process as claimed in one of the preceding claims, **characterized in that** step e) of adjusting the composition of the impregnation liquor is performed by adding water and/or acid to said impregnation liquor used in step a), so as to keep constant, throughout the process, the acidic power of the liquor separated out obtained from step b) and/or of the liquid streams withdrawn during the biomass pretreatment, notably to within ± 20%, preferably to within ± 10%.

14. The process as claimed in one of the preceding claims, **characterized in that** it comprises a step of recycling of the liquor separated out obtained from step b), of the liquid streams withdrawn during the biomass pretreatment and/or of the liquid streams withdrawn during the subsequent biomass treatment steps, for example by enzymatic hydrolysis and/or alcoholic fermentation, into the impregnation step a).

15. The process as claimed in one of the preceding claims, **characterized in that** it comprises, after the pretreatment step c), a step of treating at least a part of the pretreated biomass, by enzymatic hydrolysis, to produce said sugary liquors.

16. The process as claimed in one of the preceding claims, **characterized in that** at least one portion of the sugary liquors is subjected to an alcoholic fermentation.
